# EUROPEAN PATENT SPECIFICATION

(11) **EP 3 965 856 B1**
(45) Date of publication and mention of the grant of the patent: **21.08.2024**
(21) Application number: 20801904.2
(22) Date of filing: 30.04.2020
(51) Int. Cl.: A61M 15/00, G08B 21/00

(54) **WARNING SYSTEM, INHALER, AND METHOD FOR GENERATING WARNING**
WARNSYSTEM, INHALATOR UND VERFAHREN ZUR ERZEUGUNG EINER WARNUNG
SYSTÈME D'AVERTISSEMENT, INHALATEUR ET PROCÉDÉ DE GÉNÉRATION D'AVERTISSEMENT

(30) Priority: 07.05.2019 US 201962844221 P
(43) Date of publication of application: 16.03.2022
(73) Proprietor: Kindeva Drug Delivery L.P., Woodbury, MN 55129 (US)
(72) Inventor: GRIFFITHS, Neale F., Bracknell Berkshire RG12 8HT (GB); MOHAMMED, Parfes, Bracknell Berkshire RG12 8HT (GB); STUART, Adam, Loughborough Leicestershire LE12 6JA (GB); JOLLY, Paul H. R., Bracknell Berkshire RG12 8HT (GB)
(74) Representative: Vossius & Partner Patentanwälte Rechtsanwälte mbB
(86) International application number: PCT/IB2020/054091
(87) International publication number: WO 2020/225666

(56) References cited:
- WO-A1-2016/030521
- WO-A1-2017/112451
- WO-A1-2018/149620
- WO-A1-2020/161423
- WO-A2-2008/070516
- US-A- 5 692 492
- US-A1- 2002 189 615
- US-A1- 2016 228 657
- US-A1- 2017 087 312
- US-A1- 2019 015 609

## Description

### Technical Field

The present disclosure generally relates to inhalation devices and, more particularly, to inhalers having a warning system for generating a warning and a method of generating the warning.

### Background

Inhalers for pulmonary delivery are designed to deliver a medicament to an oral cavity of a patient. Such inhalers can include a breath-actuated trigger mechanism that delivers the medicament based on an inhalation of the patient. Based on the inhalation of the patient, a metered amount of the medicament is introduced into an orifice of the inhaler by a metering valve. The metering valve is in selective fluid communication with a fluid source, such as a canister, that holds the medicament. From the orifice, the medicament enters a mouthpiece of the inhaler.

WO 2020/161423 A1 is a document that was filed before the priority date of the present patent document and was published after the filing date of the present patent document. WO 2020/161423 A1 discloses a device for dispensing a fluid product synchronized with inhalation.

WO 2016/030521 A1 discloses an inhaler including a mouthpiece cover, a pressure sensor, a first indicator and a second indicator. The first indicator may be configured to indicate based on a state of the cover, and the second indicator may be configured to indicate based on an output of the pressure sensor. For example, when the mouthpiece cover opens, the first indicator illuminates and a dose of medication may be transferred from a reservoir to a dosing cup. The second indicator illuminates if an amount of inhaled medication reaches a predetermined threshold for successful inhalation.

Other inhalers of the prior art are shown in WO 2018/149620 A1, WO 2008/070516 A2 and WO 2017/112451 A1.

### Summary

In one aspect, the present disclosure relates to a warning system for use with an inhaler. The inhaler includes an operating mechanism that is configured to be actuated to a reset state, a primed state, and a fired state. The inhaler dispenses a metered dose of a medicament upon actuation of the operating mechanism from the primed state to the fired state. The warning system includes a control circuit that is activated upon actuation of the operating mechanism from the primed state to the fired state. The warning system also includes an alert device communicably coupled to the control circuit and configured to generate a warning. Further, the control circuit is configured to control the alert device to generate the warning until the operating mechanism has been actuated from the fired state to the reset state. The reset state is a state in which a lever is returned to a closed position after inhalation of the metered dose of the medicament by a patient, and a metering valve is returned to a deactivated position. The primed state is a state in which the lever is rotated to an open position, a spring compresses and pushes on a canister that contains the medicament, and the canister remains static. The fired state is a state in which the lever remains in the open position, and upon inhalation by the patient through a mouthpiece, energy stored in the spring depresses the canister, which activates the metering valve, and the metered dose of the medicament is released.

In another aspect, the present disclosure relates to an inhaler for dispensing a medicament. The inhaler includes an operating mechanism that is configured to be actuated to a reset state, a primed state, and a fired state. The inhaler includes a dispensing mechanism configured to dispense a metered dose of the medicament upon actuation of the operating mechanism from the primed state to the fired state. The inhaler also includes the above-described warning system.

In another aspect, the present disclosure relates to a method of generating a warning after use of an inhaler. The inhaler includes an operating mechanism that is configured to be actuated to a reset state, a primed state, and a fired state. The inhaler dispenses a metered dose of a medicament upon actuation of the operating mechanism from the primed state to the fired state. The method includes activating a control circuit upon actuation of the operating mechanism from the primed state to the fired state. The method also includes generating a warning by an alert device until the operating mechanism has been actuated from the fired state to the reset state. Further, the alert device is controlled by the control circuit. The reset state is a state in which a lever is returned to a closed position after inhalation of the metered dose of the medicament by a patient, and a metering valve is returned to a deactivated position. The primed state is a state in which the lever is rotated to an open position, a spring compresses and pushes on a canister that contains the medicament, and the canister remains static. The fired state is a state in which the lever remains in the open position, and upon inhalation by the patient through a mouthpiece, energy stored in the spring depresses the canister, which activates the metering valve, and the metered dose of the medicament is released.

### Brief Description of the Drawings

Exemplary embodiments disclosed herein may be more completely understood in consideration of the following detailed description in connection with the following figures. The figures are not necessarily drawn to scale. Like numbers used in the figures refer to like components. However, it will be understood that the use of a number to refer to a component in a given figure is not intended to limit the component in another figure labeled with the same number.
FIG. 1 is a perspective view of an inhaler in a reset state according to embodiments of the present disclosure;
FIG. 2 is a perspective view of the inhaler depicted in FIG. 1 in a primed state;
FIG. 3 is a sectional view of a dispensing mechanism associated with the inhaler depicted in FIG. 1 according to embodiments of the present disclosure;
FIG. 4 is a perspective view of an operating mechanism associated with the inhaler depicted in FIG. 1 according to embodiments of the present disclosure;
FIGS. 5 and 6 illustrate operation of the operating mechanism associated with the inhaler depicted in FIG. 1 according to embodiments of the present disclosure;
FIG. 7 is a block diagram illustrating a warning system associated with the inhaler depicted in FIG. 1 according to embodiments of the present disclosure;
FIG. 8 is a perspective view of a flange member associated with the warning system depicted in FIG. 7 according to embodiments of the present disclosure;
FIG. 9 is a sectional view of the inhaler showing the flange member according to embodiments of the present disclosure;
FIG. 10 illustrates a switch and a control circuit associated with the warning system depicted in FIG. 7 according to embodiments of the present disclosure;
FIGS. 11 and 12 are sectional views of a portion of the inhaler illustrating the flange member associated with the warning system depicted in FIG. 7 according to embodiments of the present disclosure;
FIGS. 13 and 14 illustrate different views of a pocket that receives the control circuit associated with the warning system depicted in FIG. 7 according to embodiments of the present disclosure;
FIG. 15 illustrates an alert device and the control circuit associated with the warning system depicted in FIG. 7 according to embodiments of the present disclosure;
FIG. 16 illustrates a first warning strategy implemented by the warning system depicted in FIG. 7 according to embodiments of the present disclosure;
FIG. 17 illustrates a second warning strategy implemented by the warning system depicted in FIG. 7 according to embodiments of the present disclosure; and
FIG. 18 is a flowchart for a method of generating a warning after use of the inhaler.

### Detailed Description

In the following description, reference is made to the accompanying figures that form a part thereof and in which various embodiments are depicted by way of illustration. It is to be understood that other embodiments are contemplated and may be made without departing from the scope or spirit of the present disclosure. The following detailed description, therefore, is not to be taken in a limiting sense.

The present disclosure will be described with respect to particular embodiments and with reference to certain drawings, but the disclosure is not limited thereto. The drawings described are only schematic and are non-limiting. In the drawings, the size of some of the elements may for illustrative purposes be exaggerated and not drawn to scale.

It will be understood that the terms "vertical", "horizontal", "top", "bottom", "above", "below", "left", "right" etc. as used herein refer to particular orientations of the figures and these terms are not limitations to the specific embodiments described herein.

Inhalers can comprise a canister-retaining or tubular housing portion and a tubular mouthpiece portion, the tubular mouthpiece portion being angled with respect to the tubular housing portion. An air inlet is defined proximal to at least one of an upper end and a lower end of the tubular housing portion. Further, a dispensing mechanism is disposed within the tubular housing portion that dispenses a medicament from a canister or reservoir of the inhaler. The dispensing mechanism includes a metering valve that activates during delivery of the medicament. The metering valve needs to be reset after each use to position it so that it can later be refilled from the reservoir to provide the next dose. In operation of the inhaler, a plume of the medicament is dispensed by the dispensing mechanism into the tubular mouthpiece portion and is inhaled by a patient through the tubular mouthpiece portion. However, the metering valve may not be reset directly after such use and may be susceptible to damage if left in the fired state for an extended period.

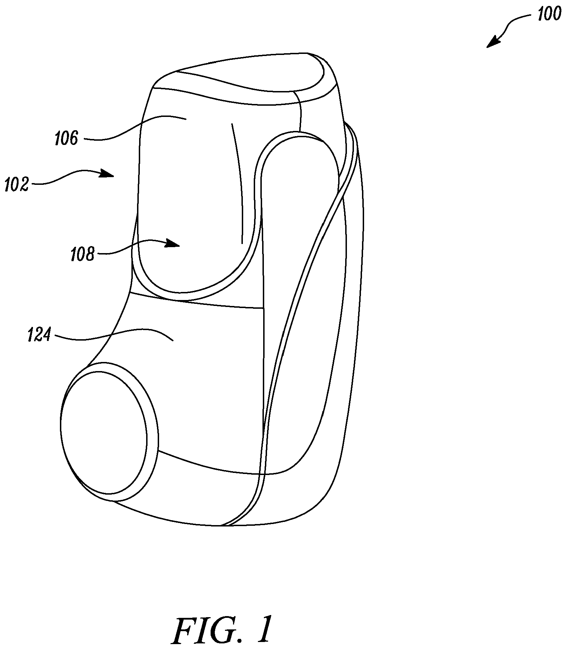

FIG. 1 illustrates a perspective view of an inhaler 100 for dispensing a medicament to a patient. The inhaler 100 may be embodied as an electronic inhaler. Further, the inhaler 100 may include an onboard power source (not depicted), such as batteries or cells, that powers various electronic components of the inhaler 100. The inhaler 100 may be embodied as a Pressurized Metered-Dose Inhaler (pMDI).

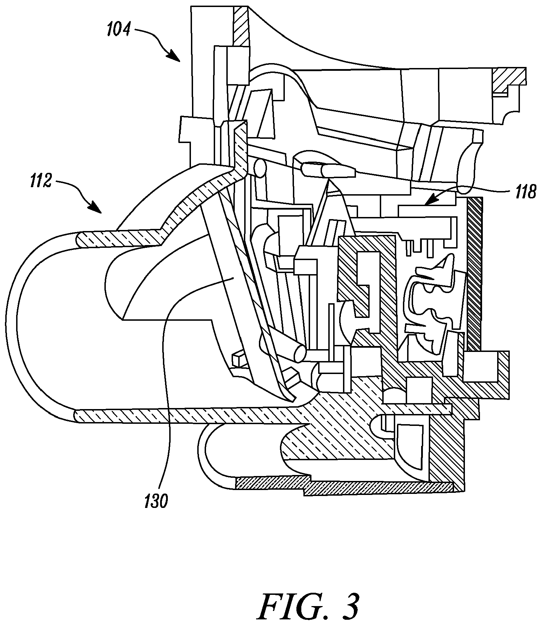

In the illustrated embodiment, the inhaler 100 is a breath-actuated inhaler. In such a case, a metering valve (not depicted) may be actuated by a pressure differential created by inhalation of a patient to automatically dispense a spray of the medicament without any manual intervention. The inhaler 100 includes an actuator housing 102 for holding the medicament. The actuator housing 102 includes a housing portion 104 (depicted in FIG. 3). The housing portion 104 may have a substantially hollow structure and is tubular in shape.

The actuator housing 102 also includes a cover member 106 that covers the housing portion 104. The cover member 106 defines an outer surface 108. The outer surface 108 may define a grip section (not depicted). The grip section is defined proximate to a bottom end of the cover member 106. The grip section allows a user to grip the inhaler 100 while using the inhaler 100. The grip section may essentially be a set of protrusions, a set of indents, or a sleeve providing a better gripping surface compared to the outer surface 108, or any other such structural arrangement. Further, the actuator housing 102 includes an air inlet (not depicted) for receiving the air flow. The air inlet may be defined at an upper end or a lower end of the actuator housing 102. The actuator housing 102 includes an air inlet cover (not depicted) that defines the air inlet. The air inlet cover may be embodied as a grille.

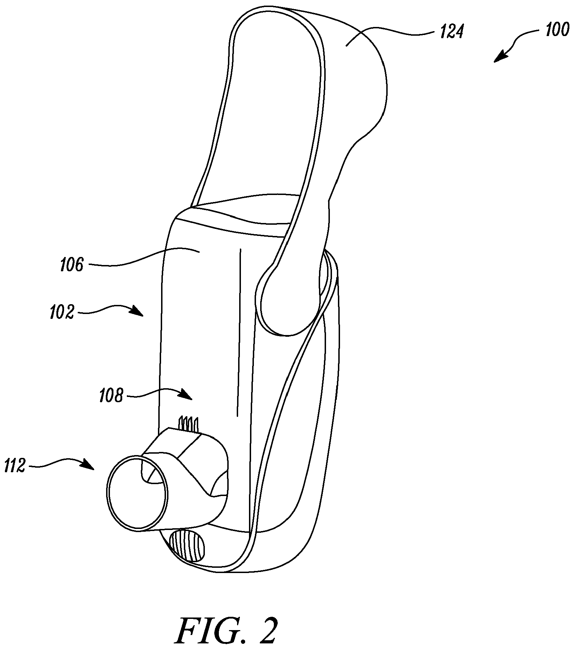

Referring to FIG. 2, the inhaler 100 includes a mouthpiece 112. The mouthpiece 112 is embodied as a generally tubular portion extending from the actuator housing 102 and may have a substantially hollow structure. The mouthpiece 112 is joined to the housing portion 104. In an example, the mouthpiece 112 is angled with respect to the actuator housing 102. The mouthpiece 112 may have a circular cross-section or a non-circular cross-section such as an elliptical or oblong cross-section. A user or the patient may put at least a part of the mouthpiece 112 into his mouth for using the inhaler 100.

A canister (not depicted) is removably received within the housing portion 104. The canister contains a fluid formulated with the medicament and may be embodied as an aerosol canister. In another embodiment, the fluid formulated with the medicament may be stored in a reservoir. The canister may have a generally cylindrical structure. The canister releases a predetermined amount of the medicament based on an actuation of a dispensing mechanism 118. Referring to FIG. 3, the dispensing mechanism 118 is configured to dispense a metered dose of the medicament as per requirements. The dispensing mechanism 118 includes the metering valve. The predetermined amount of the medicament passes through the metering valve upon activation of the metering valve. When activated, the metering valve is in fluid communication with the canister. The metering valve meters an amount of the medicament exiting the canister corresponding to a single spray pattern or spray plume. The dispensing mechanism 118 further includes a valve stem (not depicted) extending from the metering valve and a nozzle block (not depicted) having a stem socket. The nozzle block sits at a closed bottom end of the actuator housing 102. The stem socket is provided for receiving the valve stem. Further, the stem socket includes an exit orifice communicating with the mouthpiece 112 (depicted in FIG. 2) of the inhaler 100.

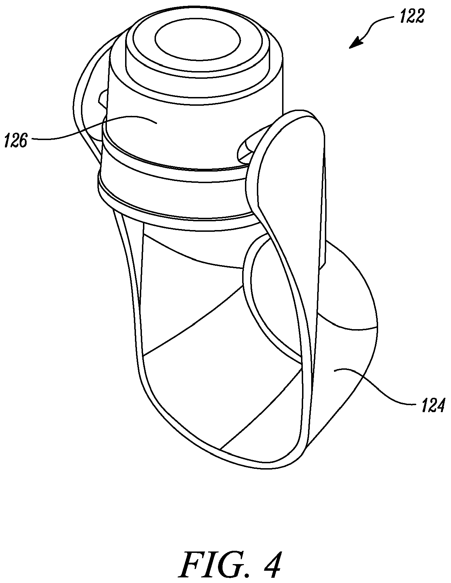

As depicted in FIG. 4, the inhaler 100 includes an operating mechanism 122. The operating mechanism 122 is configured to be actuated to a reset state, a primed state, and a fired state. The inhaler 100 dispenses the metered dose of the medicament upon actuation of the operating mechanism 122 from the primed state to the fired state. More particularly, the dispensing mechanism 118 is configured to dispense the metered dose of the medicament upon actuation of the operating mechanism 122 from the primed state to the fired state. The operating mechanism 122 is said to be in the reset state when the inhaler 100 is not in operation and the metering valve is in a deactivated or closed position. Further, the operating mechanism 122 is said to be in the fired state when the inhaler 100 is in operation and the metering valve is in an activated or open position. The primed state of the operating mechanism 122 will be explained later in this section.

The operating mechanism 122 includes a lever 124. The lever 124 is pivotable between a closed position and an open position. The operating mechanism 122 may be in the primed state or the fired state when the lever 124 is in the open position. Whereas, the operating mechanism 122 is said to be in the reset state when the lever 124 is in the closed position. Further, the lever 124 performs a function of a dust cap by isolating the mouthpiece 112 (see FIG. 2) from outside environment when the inhaler 100 is not in use. The operating mechanism 122 also includes a cylinder 126 that is received within the actuator housing 102. The cylinder 126 is coupled to the lever 124 such that an operation of the lever 124 between the closed and open positions causes the cylinder 126 to reciprocate within the actuator housing 102.

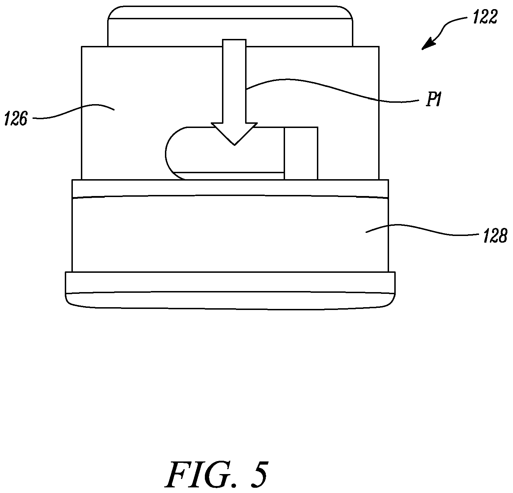

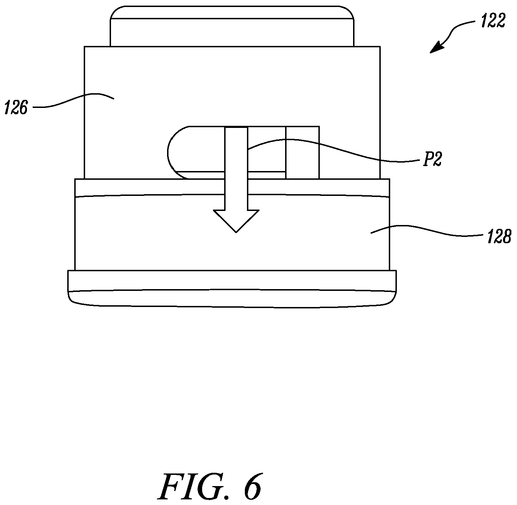

Referring to FIG. 5, the operating mechanism 122 includes a spring 128 (depicted schematically in FIG. 5) that is operatively coupled with the cylinder 126. In use, the patient may rotate the lever 124 (see FIGS. 2 and 4) to the open position and insert the exposed mouthpiece 112 into their mouth. At such an instance, the operating mechanism 122 has been actuated to the primed state. The rotation of the lever 124 causes an off-center cam (not depicted) to compress the spring 128, as illustrated by arrow "P1". The spring 128 compresses and pushes on the canister via the cylinder 126. However, the canister remains static and may be prevented from moving by a rocker (not depicted) which is held in place by a catch (not depicted). The rocker and the catch may form a part of the dispensing mechanism 118. On inhalation by the patient through the mouthpiece 112, the operating mechanism 122 is actuated to the fired state from the primed state. More particularly, during inhalation, the airflow caused by inhalation may move a vane 130 (see FIG. 3) of the dispensing mechanism 118 which releases the catch and allows the rocker to rotate. As depicted in FIG. 6, when the operating mechanism 122 is in the fired state, energy stored in the spring 128 during the priming state depresses the canister via the cylinder 126, as illustrated by arrow "P2", which in turn activates the metering valve, based on which the medicament contained within the canister is released.

Referring to FIG. 2, on inhalation by the patient through the mouthpiece 112, a pressure differential in the actuator housing 102 causes the canister to displace relative to the valve stem. The medicament contained within the metering chamber of the canister is accordingly released in response to the patient's inspiration. During the patient's inspiration, air flows from the air inlet, through the actuator housing 102. The medicament released from the canister enters this air flow. Thus, during operation of the inhaler 100, a plume of the medicament dispensed through the dispensing mechanism 118 is inhaled by the patient through the mouthpiece 112. The inhaler 100 may also include an integrated dose counter that may assist in indicating to the patient when the medicament in the canister is about to deplete and provide health monitoring data to health personnel.

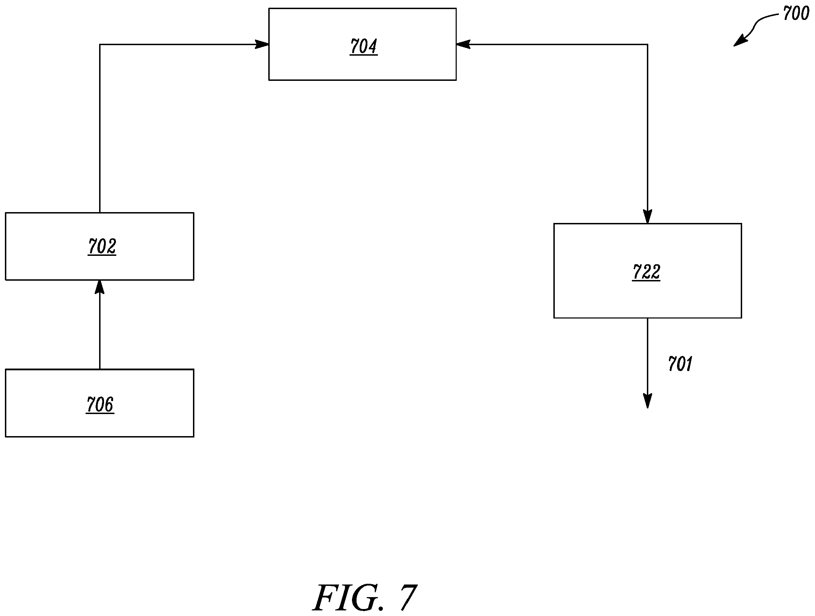

After inhalation of the metered dose of the medicament by the patient, the lever 124 needs to be returned to its closed position, such that the operating mechanism 122 (see FIG. 4) is actuated to the reset state. It should be noted that the metering valve returns to the deactivated position after the operating mechanism 122 is actuated to the reset state. Thus, to avoid potential for damage to the metering valve, the operating mechanism 122 can be actuated to the reset state by returning the lever 124 to the closed position after using the inhaler 100. As depicted in FIG. 7, the present disclosure is directed towards a warning system 700 for use with the inhaler 100. The warning system 700 may include a discreet electronic based solution for alerting the patient to actuate the operating mechanism 122 to the reset state after use in a situation where the patient might forget to reset the operating mechanism 122. The warning system 700 generates a warning 701 to alert the patient to actuate the operating mechanism 122 to the reset state. The warning 701 is at least one of an optical warning, an audio warning, and a haptic warning.

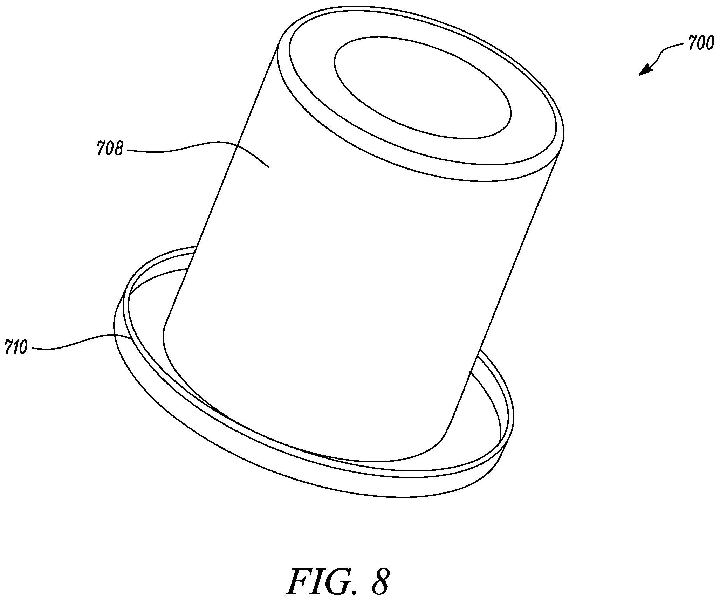

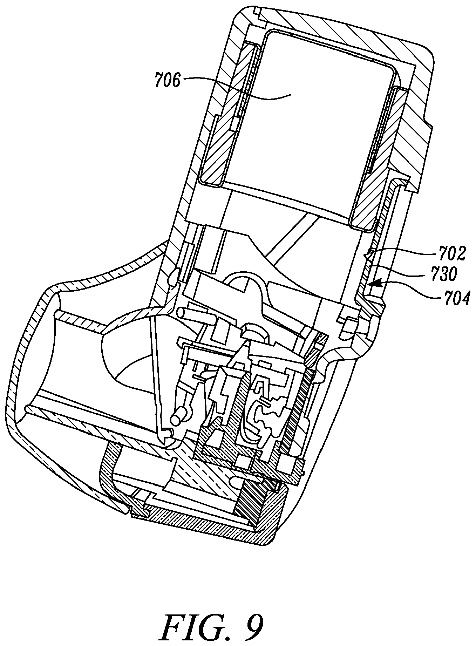

The warning system 700 includes a switch 702 that is actuated upon actuation of the operating mechanism 122 from the primed state to the fired state. The switch 702 is configured to activate a control circuit 704 upon actuation. The switch 702 may embody a micro-switch. In embodiments, as depicted in FIGS. 8 and 9, the warning system 700 includes a flange member 706 operatively coupled to the operating mechanism 122. The flange member 706 is movable upon actuation of the operating mechanism 122 from the primed state to the fired state to actuate the switch 702. The flange member 706 has a generally hollow cylindrical body 708 having a flange portion 710 that selectively contacts the switch 702 based on the movement of the flange member 706.

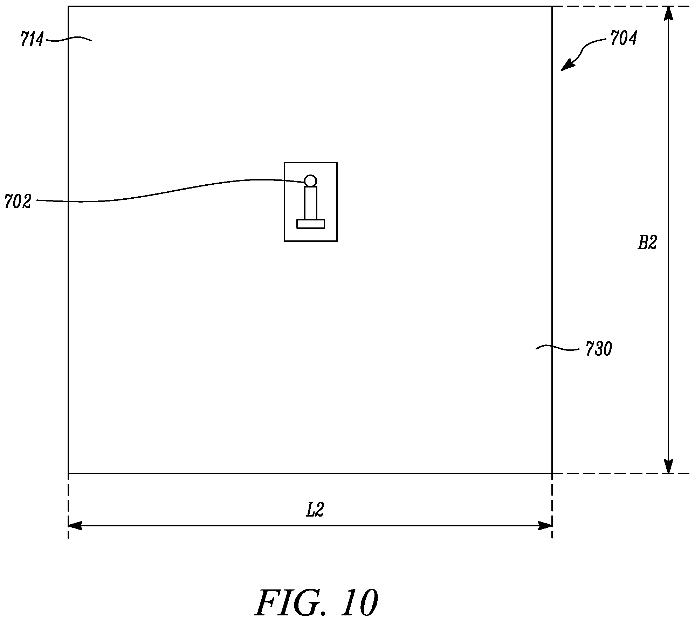

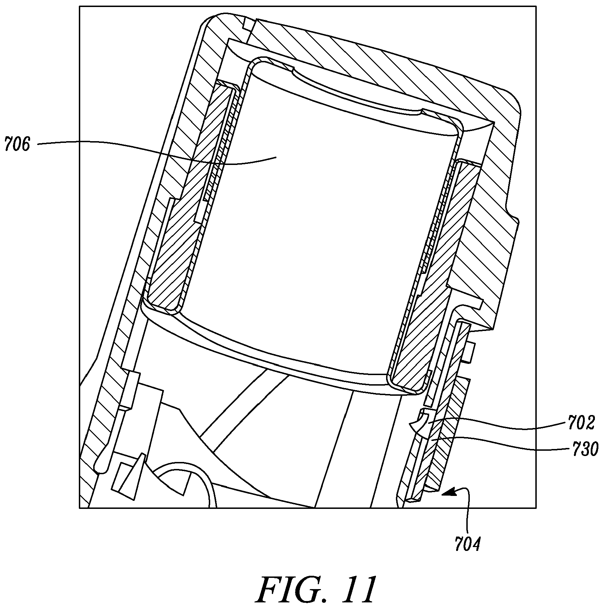

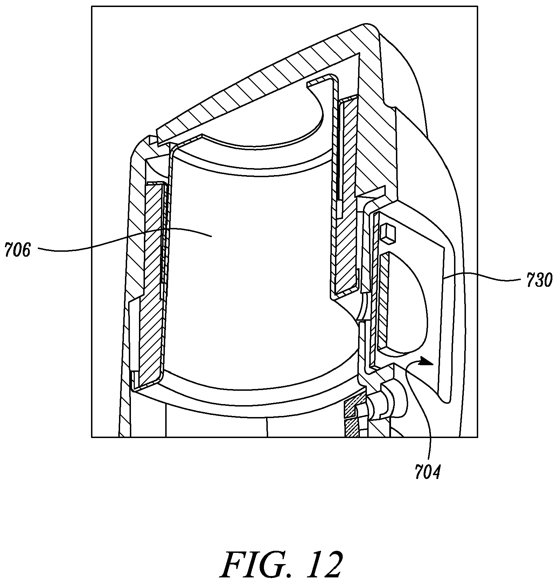

The switch 702 is operatively coupled to the control circuit 704 of the warning system 700. In the illustrated example, the control circuit 704 includes a Printed Circuit Board (PCB) 730. In an embodiment depicted in FIG. 10, the switch 702 may be positioned on the PCB 730. For example, the switch 702 may be positioned approximately at a central portion of the PCB 730 on a first surface 714 defined by the PCB 730. As depicted in FIGS. 11 and 12, the PCB 730 and the switch 702 are positioned such that the flange member 706 actuates the switch 702 based upon actuation of the operating mechanism 122 from the primed state to the fired state. The control circuit 704 is activated upon actuation of the operating mechanism 122 from the primed state to the fired state. Thus, the control circuit 704 and the switch 702 are in an activated state when the operating mechanism 122 is in the fired state. Further, the control circuit 704 is deactivated upon actuation of the operating mechanism 122 from the fired state to the reset state. The switch 702 selectively connects the control circuit 704 to a power source 732. When the switch 702 is off, the control circuit 704 is disconnected from the power source 732. Whereas, when the switch 702 is on, the control circuit 704 is connected to the power source 732, thereby activating the control circuit 704. The control circuit 704 may embody a single microprocessor or multiple microprocessors for receiving signals from components of the warning system 700. Numerous commercially available microprocessors may be configured to perform the functions of the control circuit 704. The control circuit 704 may further include a memory to store data and algorithms therein.

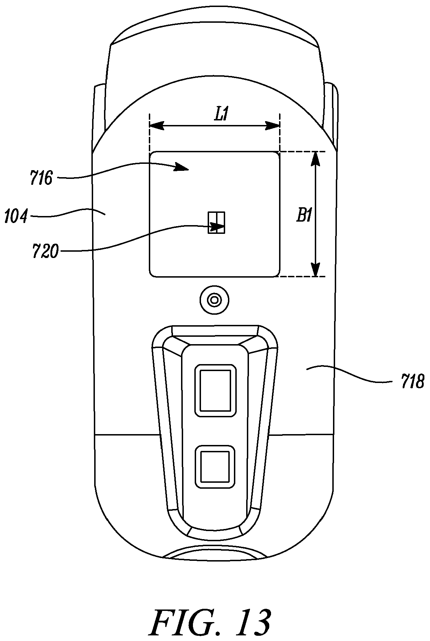

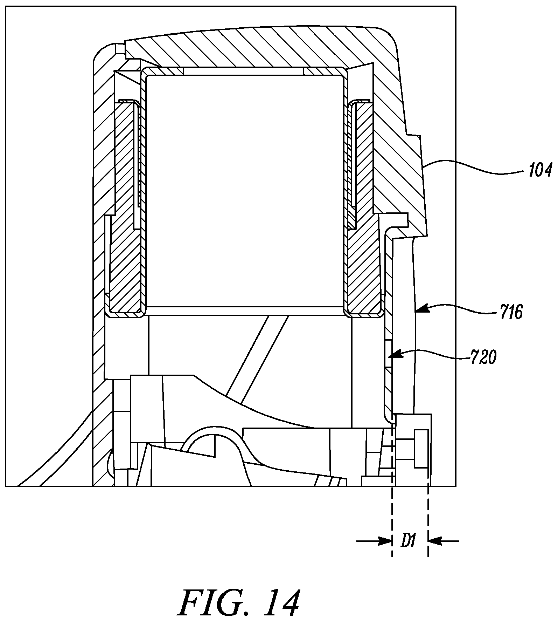

As depicted in FIGS. 13 and 14, the housing portion 104 defines a pocket 716 that receives the switch 702 and the PCB 730. The pocket 716 is defined at an outer surface 718 of the housing portion 104. The pocket 716 defines a slot 720 that receives the switch 702 such that the switch 702 projects inwards on assembly of the inhaler 100 to allow the flange member 706 to contact and actuate the switch 702. A shape and dimensions of the pocket 716 are decided based on a shape and dimensions of the PCB 730. The pocket 716 defines a length "L1" (depicted in FIG. 13), a breadth "B1" (depicted in FIG. 13), and a depth "D1" (depicted in FIG. 14) from the outer surface 718 of the housing portion 104. Further, the length "L1", the breadth "B1", and the depth "D1" is generally greater than the length "L2" (depicted in FIG. 10), the breadth "B2" (depicted in FIG. 10), and the depth (not depicted) of the PCB 730 of the control circuit 704 to receive the PCB 730 therein. The depth "D2" is defined between the first surface 714 and a second surface 728 of the PCB 730. When the inhaler 100 is assembled, the pocket 716 is covered by the cover member 106 so that sensitive components of the warning system 700 are isolated from the outside environment.

Referring to FIG. 5, the warning system 700 includes an alert device 722. The alert device 722 is communicably coupled to the control circuit 704 and is configured to generate the warning 701. The control circuit 704 is configured to control the alert device 722 to generate the warning 701 until the operating mechanism 122 has been actuated from the fired state to the reset state. Further, the control circuit 704 is configured to control the alert device 722 to generate the warning 701 after a predetermined time delay from the actuation of the operating mechanism 122 to the fired state. In some embodiments, the predetermined time delay is variable. Such a predetermined but variable time delay may be based on patient preferences and aims at avoiding false warnings.

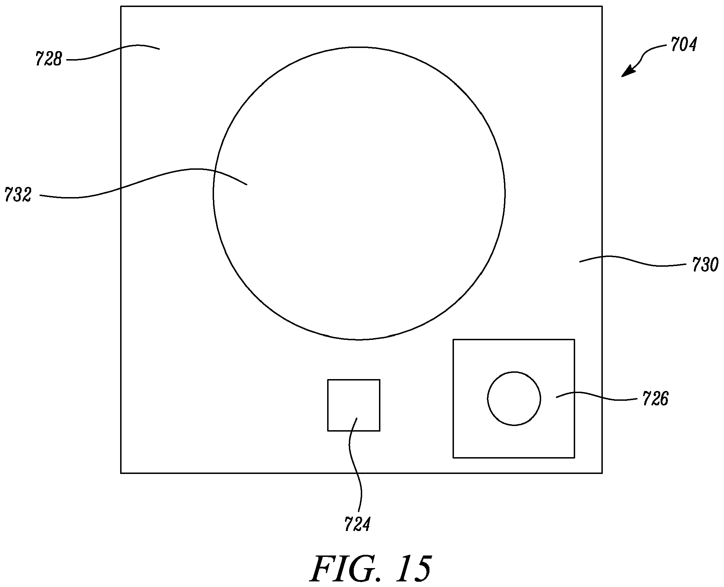

In some embodiments, the alert device 722 is at least one of an optical device, an audio device, and a haptic device. In some embodiments, the alert device 722 may include a single output device or a combination of output devices that generate the warning 701. As depicted in FIG. 15, the alert device 722 of the illustrated example includes the optical device 724 and the audio device 726. The optical device 724 is a Light Emitting Diode (LED) wherein the warning 701 is issued based on illumination of the LED. The audio device 726 is a buzzer wherein the warning 701 is issued based on a sound generated by the buzzer. Alternatively, the optical and audio devices 724, 726 may include any other known output device that issues the warning 701, without limiting the scope of the present disclosure. The optical device 724 and the audio device 726 may be positioned on the second surface 728 of the PCB 730. Further, the alert device 722 may also include the haptic device that provides a haptic vibration to the patient to issue the warning 701. In some embodiments, the audio device 726 may provide both an audio warning and a vibrational warning. It should be noted that a nature of the warning 701 may be customized based on patient preferences, and the patient may have an option to program and vary the nature of the warning 701 to achieve maximum impact in terms of alerting the patient to actuate the operating mechanism 122 to the reset state.

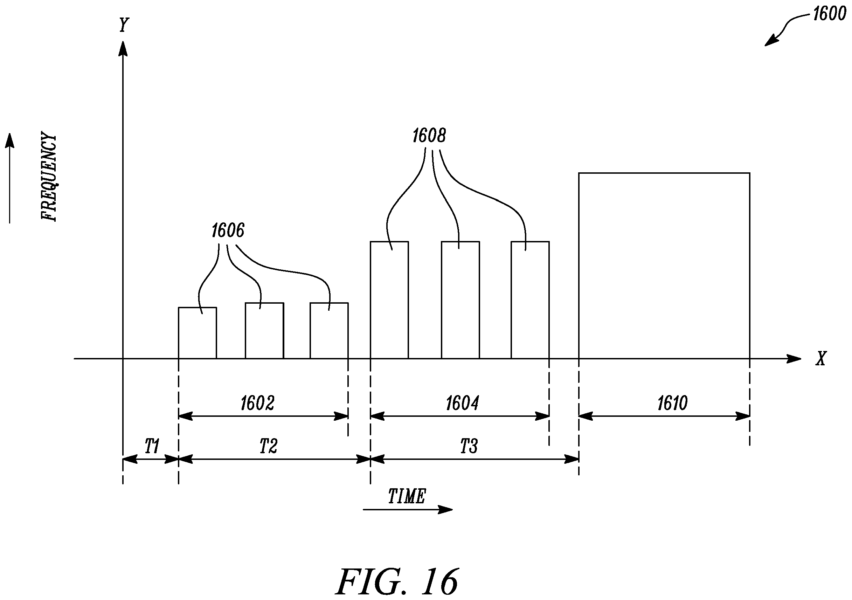

Referring now to FIG. 16, a plot 1602 illustrating a first warning strategy of the warning system 700 will be described. In an example, the control circuit 704 is configured to control the alert device 722 to generate a first warning 1602 and a second warning 1604 after the first warning 1602. In an embodiment, the first warning 1602 is generated after a first time delay "T1" from the actuation of the operating mechanism 122 to the fired state. In an example, the first time delay "T1" is approximately equal to 15 seconds. In other embodiments, the first time delay is about 5 seconds, about 10 seconds, or about 30 seconds. The first time delay "T1" is decided such that the first warning 1602 is generated only after an intended amount of the metered dose is released from the canister. In an example, the first warning 1602 comprises a plurality of first blips 1606 having a first amplitude and a first frequency. That is, each blip is a short sound with a specific frequency and they are repeated in a series having a predetermined pattern to draw attention. For example, the plurality of first blips 1606 may be embodied as pre-warning blips that are issued before the second warning 1604 is issued. The plurality of first blips 1606 may provide improved user acceptance as the blips are short and may have a relatively low frequency to bring the patient's attention to a current state of the operating mechanism 122, thus the patient can actuate the operating mechanism 122 to the reset state in a discreet manner before the second warning 1604 is issued. Thus, in such situations, the first warning 1602 issued by the warning system 700 should not distract or attract attention of other people present in the area.

In another embodiment, a second warning 1604 is generated after a second time delay "T2" from an end of the first time delay "T1". In an example, the second time delay "T2" is approximately equal to 10 seconds. In other embodiments, the second time delay is about 5 seconds, about 20 seconds, about 30 seconds, or about 1 minute. The second warning 1604 may comprise a plurality of second blips 1608 having a second amplitude and a second frequency. The second amplitude may be greater than the first amplitude and/or the second frequency may be greater than the first frequency. This may aid in drawing attention to the need to reset the operating mechanism if the first warning was not observed. In another example, the second warning 1604 comprises a continuous audio warning. The second warning 1604 may be generated until the operating mechanism 122 has been actuated from the fired state to the reset state.

The control circuit 704 is further configured to control the alert device 722 to generate a third warning 1610 after the second warning 1604. The third warning 1610 is generated after a third time delay "T3" from an end of the second time delay "T2". In an example, the third time delay "T3" is approximately equal to 10 seconds. In other embodiments, the third time delay is about 5 seconds, about 20 seconds, about 30 seconds, or about 1 minute. The third warning 1610 may comprise a continuous audio warning having a third amplitude that is greater than the second amplitude and/or a third frequency that is greater than or equal to the second amplitude. In an example, the third warning 1610 may continue until the operating mechanism 122 is actuated from the fired state to the reset state.

In another example, one or more of the first warning 1602, the second warning 1604, and the third warning 1610 are repeated for a plurality of cycles until the operating mechanism 122 has been actuated from the fired state to the reset state. Further, in some embodiments, one or more of the first frequency and the second frequency are varied across a plurality of cycles of the first and second warnings 1602, 1604. More particularly, the amplitudes of the first, second, and/or third warnings 1602, 1604, 1610 and/or the frequencies of the first and/or second warnings 1602, 1604 may be different for different cycles. In some embodiments the amplitudes and/or frequencies of the first, second, and/or third warnings may be varied within any given cycle.

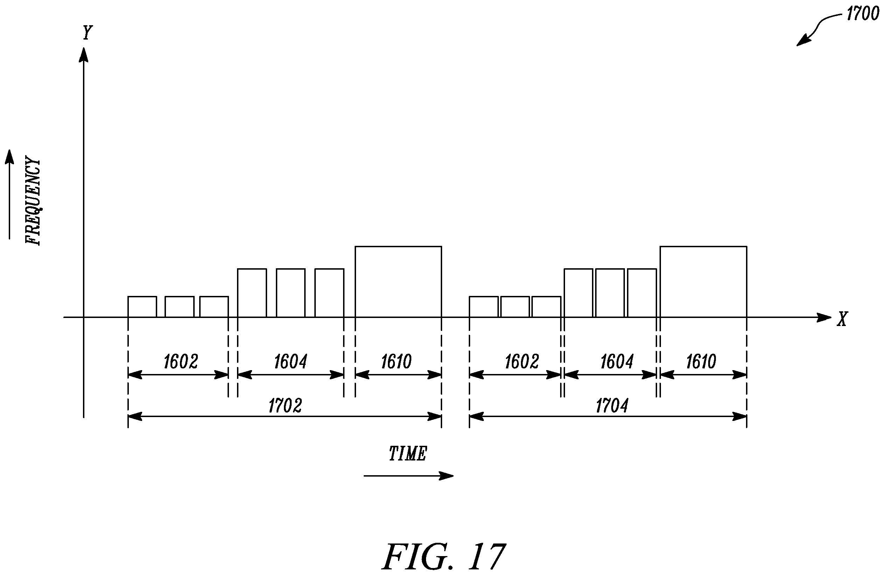

Referring now to FIG. 17, a plot 1702 illustrating a second warning strategy implemented by the warning system 700 is illustrated. As depicted, the control circuit 704 is configured to repeat each of the first, second, and third warnings 1602, 1604, 1610 for a plurality of cycles until the operating mechanism 122 has been actuated from the fired state to the reset state. In this example, each of the first, second, and third warnings 1602, 1604, 1610 is repeated for a first cycle 1702 and a second cycle 1704. However, the first, second, and third warnings 1602, 1604, 1610 may be repeated for more than two cycles, without any limitations. In some examples, a frequency of the second cycle 1704 increases after the first cycle 1702, such that the frequency of the second cycle 1704 is greater than a frequency of the first cycle 1702, and so on. Further, in some examples, the third warning 1610 may continue until the patient resets the operating mechanism 122. For example, the third warning 1610 may continue after the second cycle 1704 until the patient resets the operating mechanism 122. It should be noted that a total number of the cycles, the amplitudes of the first, second, and/or third warnings 1602, 1604, 1610, the frequencies of the first and/or second warnings 1602, 1604, and the first, second, and third time delay "T1", "T2", and "T3" may be varied based on preferences of the patient using the inhaler 100 and this information may be stored in the memory associated with the control circuit 704.

Additionally, the warning system 700 also includes the power source 732, such as a battery, to power various components of the warning system 700 such as the control circuit 704 and the alert device 722. The power source 732 may include any other power source, such as one or more button cells. Further, the warning system 700 may receive power from the power source 732 only when the operating mechanism 122 is in the primed state and the fired state, thus the warning system 700 has a longer shelf life.

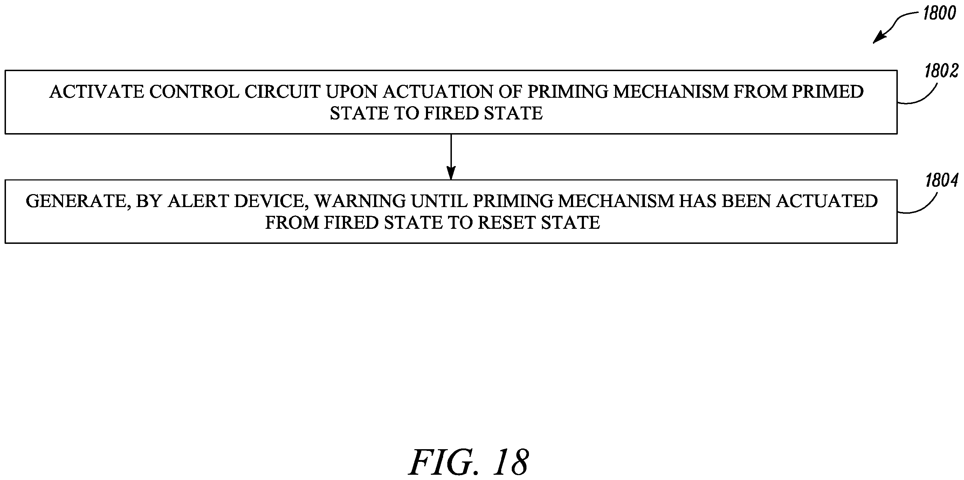

FIG. 18 illustrates a method 1800 of generating the warning 701 after use of the inhaler 100. The warning 701 is at least one of the optical warning, the audio warning, and the haptic warning. The inhaler 100 includes the operating mechanism 122 that is configured to be actuated to the reset state, the primed state, and the fired state. The inhaler 100 dispenses the metered dose of the medicament upon actuation of the operating mechanism 122 from the primed state to the fired state.

At step 1802, the control circuit 704 is activated upon actuation of the operating mechanism 122 from the primed state to the fired state. More particularly, the flange member 706 moves upon actuation of the operating mechanism 122 from the primed state to the fired state to actuate the switch 702. Thus, the switch 702 is actuated upon actuation of the operating mechanism 122 from the primed state to the fired state, wherein the switch 702 is configured to activate the control circuit 704 upon actuation. More particularly, the switch 702 selectively connects the control circuit 704 to the power source 732. When the switch 702 is off, the control circuit 704 is disconnected from the power source 732. When the switch 702 is on, the power source 732 is connected to the control circuit 704, thereby activating the control circuit 704.

At step 1804, the alert device 722 generates the warning 701 until the operating mechanism 122 has been actuated from the fired state to the reset state. Further, the control circuit 704 is deactivated upon actuation of the operating mechanism 122 from the fired state to the reset state. The alert device 722 is controlled by the control circuit 704. The warning 701 is generated after the predetermined time delay from the actuation of the operating mechanism 122 to the fired state. The predetermined time delay is variable.

In one example, generating the warning 701 includes generating of the first warning 1602 and the second warning 1604. The first warning 1602 includes the plurality of blips and the second warning 1604 includes the continuous audio warning. The second warning 1604 is generated until the operating mechanism 122 has been actuated from the fired state to the reset state. Further, the first warning 1602 is generated after the first time delay "T1" from the actuation of the operating mechanism 122 to the fired state. The first warning 1602 includes the plurality of first blips having the first amplitude and the first frequency. Further, the second warning 1604 is generated after the second time delay "T2" from the end of the first time delay "T1". The second warning 1604 includes the plurality of second blips having the second amplitude and the second frequency, the second amplitude being greater than the first amplitude, and the second frequency being greater than the first frequency. In one example, one or more of the first frequency and the second frequency are varied across the plurality of cycles.

Additionally, generating the warning 701 includes generating the third warning 1610 after the second warning 1604. The third warning 1610 is generated after the third time delay "T3" from the end of the second time delay "T2". The third warning 1610 includes the continuous audio warning having the third amplitude greater than the second amplitude. In one example, one or more of the first warning 1602, the second warning 1604, and the third warning 1610 are repeated for the plurality of cycles until the operating mechanism 122 has been actuated from the fired state to the reset state.

The warning system 700 described herein provides a low-cost and easy to implement solution for minimizing the possibility of damage to the metering valve which might occur if the operating mechanism 122 is not actuated to the reset state. Further, the warning system 700 may be implemented in a way that does not increase a size of the inhaler 100 as the components of the warning system 700 may be compact and accommodated within the actuator housing 102 without increasing a size of the actuator housing 102.

The warning system 700 described above may form a part of an electronic inhaler such as described in International Patent Application Publication WO 2017/112400, "Medicinal Inhalers." The teachings of the present disclosure can be applied to both electronic adherence monitoring addon and integrated add-in inhalation devices. This disclosure helps in providing valuable feedback to both the patients and the health care professionals thereby improving adherence monitoring. For example, a functionality of the warning system 700 may be expanded to record a dose event with a timestamp that may be communicated to a data collection infrastructure, which in turn may assist in improving patient outcome and compliance.

Unless otherwise indicated, all numbers expressing feature sizes, amounts, and physical properties used in the specification and claims are to be understood as being modified by the term "about". Accordingly, unless indicated to the contrary, the numerical parameters set forth in the foregoing specification and attached claims are approximations that can vary depending upon the desired properties sought to be obtained by those skilled in the art utilizing the teachings disclosed herein.

Although specific embodiments have been illustrated and described herein, it will be appreciated by those of ordinary skill in the art that a variety of alternate and/or equivalent implementations can be substituted for the specific embodiments depicted and described without departing from the scope of the present claims. This application is intended to cover any adaptations or variations of the specific embodiments discussed herein. Therefore, it is intended that this disclosure be limited only by the claims.

## Claims

1. A warning system (700) for use with an inhaler (100), the inhaler having an operating mechanism (122) that is configured to be actuated to a reset state, a primed state, and a fired state, the inhaler dispensing (100) a metered dose of a medicament upon actuation of the operating mechanism from the primed state to the fired state, the warning system (700) comprising:
a control circuit (704) that is activated upon actuation of the operating mechanism (122) from the primed state to the fired state; and
an alert device (722) communicably coupled to the control circuit (704) and configured to generate a warning (701),
wherein the control circuit (704) is configured to control the alert device (722) to generate the warning (701) until the operating mechanism (122) has been actuated from the fired state to the reset state,
wherein the reset state is a state in which a lever (124) is returned to a closed position after inhalation of the metered dose of the medicament by a patient, and a metering valve is returned to a deactivated position,
wherein the primed state is a state in which the lever (124) is rotated to an open position, a spring (128) compresses and pushes on a canister that contains the medicament, and the canister remains static, and
wherein the fired state is a state in which the lever (124) remains in the open position, and upon inhalation by the patient through a mouthpiece, energy stored in the spring (128) depresses the canister, which activates the metering valve, and the metered dose of the medicament is released.

2. The warning system (700) of claim 1, wherein the control circuit (704) is further configured to control the alert device (722) to generate the warning (701) after a predetermined time delay from the actuation of the operating mechanism (122) from the primed state to the fired state.

3. The warning system (700) of claim 1, wherein the control circuit (704) is deactivated upon actuation of the operating mechanism (122) from the fired state to the reset state.

4. The warning system (700) of claim 1, further comprising a switch (702) that is actuated upon actuation of the operating mechanism (122) from the primed state to the fired state, wherein the switch (702) is configured to activate the control circuit (704) upon actuation.

5. The warning system (700) of claim 1, wherein the control circuit (704) is further configured to control the alert device (722) to generate a first warning (1602) and a second warning (1604) after the first warning (1602).

6. The warning system (700) of claim 5, wherein the first warning (1602) comprises a plurality of blips.

7. The warning system (700) of claim 5, wherein the second warning (1604) comprises a continuous audio warning.

8. The warning system (700) of claim 5, wherein the second warning (1604) is generated until the operating mechanism (122) has been actuated from the fired state to the reset state.

9. The warning system (700) of claim 5, wherein the first warning (1602) is generated after a first time delay (T1) from the actuation of the operating mechanism to the fired state.

10. The warning system (700) of claim 9, wherein the second warning (1604) is generated after a second time delay (T2) from an end of the first time delay.

11. The warning system (700) of claim 10, wherein the control circuit (704) is further configured to control the alert device to generate a third warning (1610) after the second warning (1604).

12. The warning system (700) of claim 11, wherein the third warning (1610) is generated after a third time delay (T3) from an end of the second time delay (T2).

13. The warning system (700) of claim 11, wherein:
the first warning (1602) comprises a plurality of first blips having a first amplitude and a first frequency;
the second warning (1604) comprises a plurality of second blips having a second amplitude and a second frequency, the second amplitude being greater than the first amplitude, and the second frequency being greater than the first frequency; and
the third warning (1610) comprises a continuous audio warning having a third amplitude greater than the second amplitude.

14. An inhaler (100) for dispensing a medicament, the inhaler (100) comprising:
an operating mechanism (122) that is configured to be actuated to a reset state, a primed state, and a fired state;
a dispensing mechanism (118) configured to dispense a metered dose of the medicament upon actuation of the operating mechanism (122) from the primed state to the fired state; and
the warning system (700) of claim 1.

15. A method of generating (1800) a warning (701) after use of an inhaler (100), the inhaler (100) having an operating mechanism (122) that is configured to be actuated to a reset state, a primed state, and a fired state, the inhaler dispensing a metered dose of a medicament upon actuation of the operating mechanism from the primed state to the fired state, the method comprising:
activating (1802) a control circuit (704) upon actuation of the operating mechanism (122) from the primed state to the fired state; and
generating (1804), by an alert device (722), a warning (701) until the operating mechanism (122) has been actuated from the fired state to the reset state, wherein the alert device (722) is controlled by the control circuit (704),
wherein the reset state is a state in which a lever (124) is returned to a closed position after inhalation of the metered dose of the medicament by a patient, and a metering valve is returned to a deactivated position,
wherein the primed state is a state in which the lever (124) is rotated to an open position, a spring (128) compresses and pushes on a canister that contains the medicament, and the canister remains static, and
wherein the fired state is a state in which the lever (124) remains in the open position, and upon inhalation by the patient through a mouthpiece, energy stored in the spring (128) depresses the canister, which activates the metering valve, and the metered dose of the medicament is released.

## Patentansprüche

1. Warnsystem (700) zur Verwendung mit einem Inhalator (100), wobei der Inhalator einen Bedienungsmechanismus (122) aufweist, der so konfiguriert ist, dass er in einen Rücksetzzustand, einen Vorbereitungszustand und einen Abgabezustand betätigt werden kann, wobei der Inhalator (100) bei Betätigung des Bedienungsmechanismus aus dem Vorbereitungszustand in den Abgabezustand eine Dosierung eines Medikaments abgibt, wobei das Warnsystem (700) aufweist:
eine Steuerschaltung (704), die bei Betätigung des Bedienungsmechanismus (122) aus dem Vorbereitungszustand in den Abgabezustand aktiviert wird; und
eine Warnvorrichtung (722), die kommunikationsfähig mit der Steuerschaltung (704) gekoppelt und konfiguriert ist, eine Warnung (701) zu erzeugen,
wobei die Steuerschaltung (704) konfiguriert ist, die Warnvorrichtung (722) zu steuern, die Warnung (701) zu erzeugen, bis der Bedienungsmechanismus (122) aus dem Abgabezustand in den Rücksetzzustand betätigt worden ist,
wobei der Rücksetzzustand ein Zustand ist, in dem ein Hebel (124) nach der Inhalation der Dosierung des Medikaments durch einen Patienten in eine geschlossene Position zurückgebracht wird und ein Dosierventil in eine deaktivierte Position zurückgebracht wird,
wobei der Vorbereitungszustand ein Zustand ist, in dem der Hebel (124) in eine offene Position gedreht wird, eine Feder (128) zusammengedrückt wird und auf einen Behälter drückt, der das Medikament enthält, und der Behälter statisch bleibt, und
wobei der Abgabezustand ein Zustand ist, in dem der Hebel (124) in der offenen Position verbleibt und bei der Inhalation durch den Patienten durch ein Mundstück die in der Feder (128) gespeicherte Energie den Behälter herunterdrückt, wodurch das Dosierventil aktiviert und die Dosierung des Medikaments freigesetzt wird.

2. Warnsystem (700) nach Anspruch 1, wobei die Steuerschaltung (704) ferner konfiguriert ist, die Warnvorrichtung (722) zu steuern, die Warnung (701) nach einer vorbestimmten Zeitverzögerung ab der Betätigung des Bedienungsmechanismus (122) vom Vorbereitungszustand zum Abgabezustand zu erzeugen.

3. Warnsystem (700) nach Anspruch 1, wobei die Steuerschaltung (704) bei Betätigung des Bedienungsmechanismus (122) aus dem Abgabezustand in den Rücksetzzustand deaktiviert wird.

4. Warnsystem (700) nach Anspruch 1, das ferner einen Schalter (702) aufweist, der bei Betätigung des Bedienungsmechanismus (122) aus dem Vorbereitungszustand in den Abgabezustand betätigt wird, wobei der Schalter (702) konfiguriert ist, bei Betätigung die Steuerschaltung (704) zu aktivieren.

5. Warnsystem (700) nach Anspruch 1, wobei die Steuerschaltung (704) ferner konfiguriert ist, die Warnvorrichtung (722) zu steuern, eine erste Warnung (1602) und eine zweite Warnung (1604) nach der ersten Warnung (1602) zu erzeugen.

6. Warnsystem (700) nach Anspruch 5, wobei die erste Warnung (1602) mehrere Signaltöne aufweist.

7. Warnsystem (700) nach Anspruch 5, wobei die zweite Warnung (1604) eine kontinuierliche Audiowarnung aufweist.

8. Warnsystem (700) nach Anspruch 5, wobei die zweite Warnung (1604) so lange erzeugt wird, bis der Bedienungsmechanismus (122) aus dem Abgabezustand in den Rücksetzzustand gebracht worden ist.

9. Warnsystem (700) nach Anspruch 5, wobei die erste Warnung (1602) nach einer ersten Zeitverzögerung (T1) nach der Betätigung des Bedienungsmechanismus in den Abgabezustand erzeugt wird.

10. Warnsystem (700) nach Anspruch 9, wobei die zweite Warnung (1604) nach einer zweiten Zeitverzögerung (T2) nach einem Ende der ersten Zeitverzögerung erzeugt wird.

11. Warnsystem (700) nach Anspruch 10, wobei die Steuerschaltung (704) ferner konfiguriert ist, die Warnvorrichtung zu steuern, nach der zweiten Warnung (1604) eine dritte Warnung (1610) zu erzeugen.

12. Warnsystem (700) nach Anspruch 11, wobei die dritte Warnung (1610) nach einer dritten Zeitverzögerung (T3) nach einem Ende der zweiten Zeitverzögerung (T2) erzeugt wird.

13. Warnsystem (700) nach Anspruch 11, wobei:
die erste Warnung (1602) mehrere erste Signaltöne mit einer ersten Amplitude und einer ersten Frequenz aufweist;
die zweite Warnung (1604) mehrere zweite Signaltöne mit einer zweiten Amplitude und einer zweiten Frequenz aufweist, wobei die zweite Amplitude größer ist als die erste Amplitude und die zweite Frequenz höher ist als die erste Frequenz; und
die dritte Warnung (1610) eine kontinuierliche Audiowarnung mit einer dritten Amplitude aufweist, die größer ist als die zweite Amplitude.

14. Inhalator (100) zur Abgabe eines Medikaments, wobei der Inhalator (100) aufweist:
einen Bedienungsmechanismus (122), der konfiguriert ist, in einen Rücksetzzustand, einen Vorbereitungszustand und einen Abgabezustand betätigt zu werden;
einen Abgabemechanismus (118), der konfiguriert ist, eine Dosierung des Medikaments bei Betätigung des Bedienungsmechanismus (122) aus dem Vorbereitungszustand in den Abgabezustand abzugeben; und
das Warnsystem (700) des Anspruchs 1.

15. Verfahren zum Erzeugen (1800) einer Warnung (701) nach der Verwendung eines Inhalators (100), wobei der Inhalator (100) einen Bedienungsmechanismus (122) aufweist, der konfiguriert ist, in einen Rücksetzzustand, einen Vorbereitungszustand und einen Abgabezustand betätigt zu werden, wobei der Inhalator eine Dosierung eines Medikaments bei der Betätigung des Bedienungsmechanismus vom Vorbereitungszustand in den Abgabezustand abgibt, wobei das Verfahren aufweist:
Aktivieren (1802) einer Steuerschaltung (704) bei Betätigung des Bedienungsmechanismus (122) aus dem Vorbereitungszustand in den Abgabezustand; und
Erzeugen (1804) einer Warnung (701) durch eine Warnvorrichtung (722), bis der Bedienungsmechanismus (122) vom Abgabezustand in den Rücksetzzustand betätigt worden ist, wobei die Warnvorrichtung (722) durch die Steuerschaltung (704) gesteuert wird,
wobei der Rücksetzzustand ein Zustand ist, in dem ein Hebel (124) nach der Inhalation der Dosierung des Medikaments durch einen Patienten in eine geschlossene Position zurückgebracht wird und ein Dosierventil in eine deaktivierte Position zurückgebracht wird,
wobei der Vorbereitungszustand ein Zustand ist, in dem der Hebel (124) in eine offene Position gedreht wird, eine Feder (128) zusammengedrückt wird und auf einen Behälter drückt, der das Medikament enthält, und der Behälter statisch bleibt, und
wobei der Abgabezustand ein Zustand ist, in dem der Hebel (124) in der offenen Position verbleibt und bei der Inhalation durch den Patienten durch ein Mundstück die in der Feder (128) gespeicherte Energie den Behälter herunterdrückt, wodurch das Dosierventil aktiviert und die Dosierung des Medikaments freigesetzt wird.

## Revendications

1. Système d'avertissement (700) à utiliser avec un inhalateur (100), ledit inhalateur ayant un mécanisme de fonctionnement (122) prévu pour être actionné dans un état de réinitialisation, un état d'amorçage et un état de déclenchement, ledit inhalateur administrant (100) une dose mesurée d'un médicament par actionnement du mécanisme de fonctionnement de l'état d'amorçage à l'état de déclenchement, ledit système d'avertissement (700) comprenant :
un circuit de commande (704) activé par actionnement du mécanisme de fonctionnement (122) de l'état d'amorçage à l'état de déclenchement ; et
un dispositif d'alerte (722) relié de manière communicante au circuit de commande (704) et prévu pour générer un avertissement (701),
où le circuit de commande (704) est prévu pour commander le dispositif d'alerte (722) afin de générer l'avertissement (701) jusqu'à ce que le mécanisme de fonctionnement (122) ait été actionné de l'état d'amorçage à l'état de réinitialisation,
où l'état de réinitialisation est un état où un levier (124) est rappelé vers une position de fermeture après inhalation de la dose mesurée du médicament par un patient, et une valve de dosage est ramenée en position désactivée,
où l'état d'amorçage est un état où le levier (124) est tourné en position d'ouverture, un ressort (128) comprime et appuie sur un flacon contenant le médicament, et le flacon reste statique, et
où l'état de déclenchement est un état où le levier (124) reste en position d'ouverture, et où, par inhalation par le patient par un embout buccal, l'énergie accumulée dans le ressort (128) enfonce le flacon, ce qui active la valve de dosage, et la dose mesurée du médicament est libérée.

2. Système d'avertissement (700) selon la revendication 1, où le circuit de commande (704) est en outre prévu pour commander le dispositif d'alerte (722) afin de générer l'avertissement (701) après un délai prédéterminé à partir de l'actionnement du mécanisme de fonctionnement (122) de l'état d'amorçage à l'état de déclenchement.

3. Système d'avertissement (700) selon la revendication 1, où le circuit de commande (704) est désactivé par actionnement du mécanisme de fonctionnement (122) de l'état d'amorçage à l'état de réinitialisation.

4. Système d'avertissement (700) selon la revendication 1, comprenant en outre un commutateur (702) actionné par actionnement du mécanisme de fonctionnement (122) de l'état d'amorçage à l'état de déclenchement, ledit commutateur (702) étant prévu pour activer le circuit de commande (704) lors de l'actionnement.

5. Système d'avertissement (700) selon la revendication 1, où le circuit de commande (704) est en outre prévu pour commander le dispositif d'alerte (722) afin de générer un premier avertissement (1602) et un deuxième avertissement (1604) consécutif au premier avertissement (1602).

6. Système d'avertissement (700) selon la revendication 5, où le premier avertissement (1602) comprend une pluralité de bips.

7. Système d'avertissement (700) selon la revendication 5, où le deuxième avertissement (1604) comprend une alerte audio continue.

8. Système d'avertissement (700) selon la revendication 5, où le deuxième avertissement (1604) est généré jusqu'à ce que le mécanisme de fonctionnement (122) ait été actionné de l'état de déclenchement à l'état de réinitialisation.

9. Système d'avertissement (700) selon la revendication 5, où le premier avertissement (1602) est généré après un premier délai (T1) entre l'actionnement du mécanisme de fonctionnement et l'état de déclenchement.

10. Système d'avertissement (700) selon la revendication 9, où le deuxième avertissement (1604) est généré après un deuxième délai (T2) après écoulement du premier délai.

11. Système d'avertissement (700) selon la revendication 10, où le circuit de commande (704) est en outre prévu pour commander le dispositif d'alerte afin de générer un troisième avertissement (1610) après le deuxième avertissement (1604).

12. Système d'avertissement (700) selon la revendication 11, où le troisième avertissement (1610) est généré après un troisième délai (T3) après écoulement du deuxième délai (T2).

13. Système d'avertissement (700) selon la revendication 11, où :
le premier avertissement (1602) comprend une pluralité de premiers bips ayant une première amplitude et une première fréquence ;
le deuxième avertissement (1604) comprend une pluralité de deuxièmes bips ayant une deuxième amplitude et une deuxième fréquence, la deuxième amplitude étant supérieure à la première amplitude, et la deuxième fréquence étant supérieure à la première fréquence ; et
le troisième avertissement (1610) comprend un avertissement audio continu ayant une troisième amplitude supérieure à la deuxième amplitude.

14. Inhalateur (100) pour l'administration d'un médicament, ledit inhalateur (100) comprenant :
un mécanisme de fonctionnement (122) prévu pour être actionné dans un état de réinitialisation, un état d'amorçage et un état de déclenchement ;
un mécanisme d'administration (118) prévu pour administrer une dose mesurée du médicament par actionnement du mécanisme de fonctionnement (122) de l'état d'amorçage à l'état de déclenchement ; et
le système d'avertissement (700) selon la revendication 1.

15. Procédé de génération (1800) d'un avertissement (701) après utilisation d'un inhalateur (100), ledit inhalateur (100) ayant un mécanisme de fonctionnement (122) prévu pour être actionné dans un état de réinitialisation, un état d'amorçage et un état de déclenchement, ledit inhalateur administrant une dose mesurée d'un médicament par actionnement du mécanisme de fonctionnement de l'état d'amorçage à l'état de déclenchement, ledit procédé comprenant :
l'activation (1802) d'un circuit de commande (704) par actionnement du mécanisme de fonctionnement (122) de l'état d'amorçage à l'état de déclenchement ; et
la génération (1804), par un dispositif d'alerte (722), d'un avertissement (701) jusqu'à ce que le mécanisme de fonctionnement (122) ait été actionné de l'état d'amorçage à l'état de réinitialisation, le dispositif d'alerte (722) étant commandé par le circuit de commande (704),
où l'état de réinitialisation est un état où un levier (124) est rappelé vers une position de fermeture après inhalation de la dose mesurée du médicament par un patient, et une valve de dosage est ramenée en position désactivée,
où l'état d'amorçage est un état où le levier (124) est tourné en position d'ouverture, un ressort (128) comprime et appuie sur un flacon contenant le médicament, et le flacon reste statique, et
où l'état de déclenchement est un état où le levier (124) reste en position d'ouverture, et où, par inhalation par le patient par un embout buccal, l'énergie accumulée dans le ressort (128) enfonce le flacon, ce qui active la valve de dosage, et la dose mesurée du médicament est libérée.
